(19) Europäisches Patentamt
European Patent Office
Office européen des brevets

(11) Publication number: **0 041 295**
**B1**

# (12) EUROPEAN PATENT SPECIFICATION

(45) Date of publication of patent specification: **04.01.84**

(51) Int. Cl.³: **C 07 C 93/06** //C07C43/168, C07D303/24

(21) Application number: **81200558.5**

(22) Date of filing: **22.05.81**

## (54) Process for the preparation of 1-isopropylamino-3-(4-(2-methoxy-ethyl)-phenoxy)-2-propanol.

(30) Priority: **26.05.80 FI 801680**

(43) Date of publication of application:
**09.12.81 Bulletin 81/49**

(45) Publication of the grant of the patent:
**04.01.84 Bulletin 84/1**

(84) Designated Contracting States:
**AT CH IT LI NL SE**

(56) References cited:
**GB - A - 1 308 106**
**NL - A - 7 902 407**
**NL - A - 7 907 207**

**CHEMICAL ABSTRACTS, vol. 77, no. 11, 11th September 1972, page 469, no. 75109r Columbus, Ohio, U.S.A. N. SAKURA et al.: "Catalytic hydrogenolyses of benzylidene compounds bound to two oxygens, two nitrogens, and both oxygen and nitrogen"**

(73) Proprietor: **Aktiebolaget Hässle**
**Kärragatan 5**
**S-431 83 Mölndal (SE)**

(72) Inventor: **Koskenniska, Lasse Antero**
**Liistetie 4 A 2**
**SF-90650 Oulu 65 (FI)**
Inventor: **Karjalainen, Arto Johannes**
**Myllyojantie 13 H 37**
**SF-90650 Oulu 65 (FI)**
Inventor: **Toivola, Reijo Juhani**
**Kuivastie 1 A 1**
**SF-90500 Oulu 50 (FI)**

(74) Representative: **Wurm, Bengt Runio et al,**
**Patent and Trade Mark Department AB ASTRA**
**S-151 85 Södertälje (SE)**

**EP 0 041 295 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of 1-isopropylamino-3-[4-(2-methoxyethyl)-phenoxy]-2-propanol

This invention relates to a new process for the preparation of 1-isopropylamino-3-[4-(2-methoxyethyl)-phenoxy]-2-propanol or metoprolol (I) and the acid addition salts thereof.

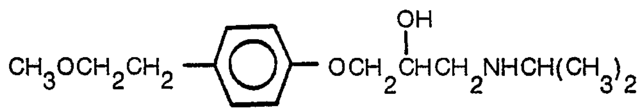

(I)

Metoprolol is a therapeutically valuable so-called $\beta$-adrenergic blocker. Most of the $\beta$-blockers have the drawback of blocking in addition to the $\beta$-receptors of the heart also the $\beta$-receptors of the blood vessels and the lungs. Contrary to this, metoprolol is heart specific and thus an important drug in the therapy of heart diseases.

The preparation of metoprolol is known from e.g. the Swedish patent No. 354 851, in which 12 processes for its preparation are described. These processes all relate to methods for the synthesis of the isopropylamino side-chain.

As starting material in the present invention is used the known and easily prepared sodium salt of 4-(methoxyethanoyl)phenol (II).

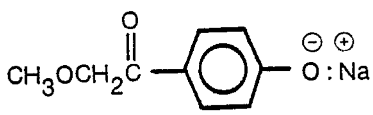

(II)

This is reduced in the first step to 4-(1-hydroxy-2-methoxyethyl)-phenol (III).

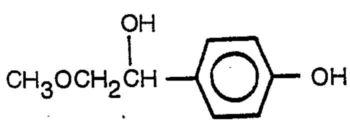

(III)

By heating a mixture of this hydroxyphenol (III) and methanol in the presence of an acid catalyst 4-(1,2-dimethoxyethyl)phenol (IV) is formed. At corresponding conditions in ethanol is formed 4-(1-ethoxy-2-methoxyethyl)phenol (V).

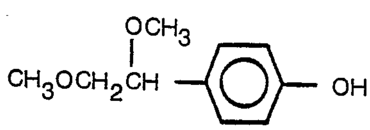

IV

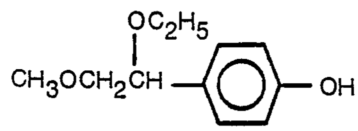

V

When the methoxyphenol (IV) and the ethoxyphenol (V) are reacted at alkaline conditions with epichlorohydrine 1,2-epoxy-3-[4-(1,2-dimethoxyethyl)phenoxy]propane (VI) and 1,2-epoxy-3-[4-(1-ethoxy-2-methoxyethyl)phenoxy]propane (VII) are obtained.

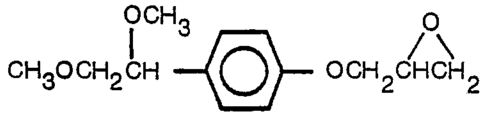

VI

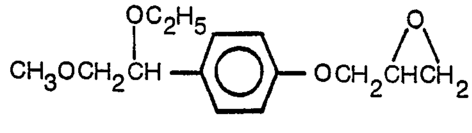

VII

When the methoxyepoxide (VI) and the ethoxyepoxide (VII) are reacted with isopropylamine, 1-isopropylamino-3-[4-(1,2-dimethoxyethyl)phenoxy]-2-propanol (VIII) and 1-isopropylamino-3-[4-(1-ethoxy-2-methoxyethyl)phenoxy]-2-propanol (IX) are formed.

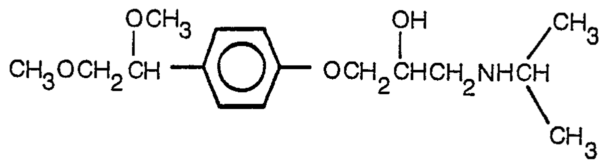

(VIII)

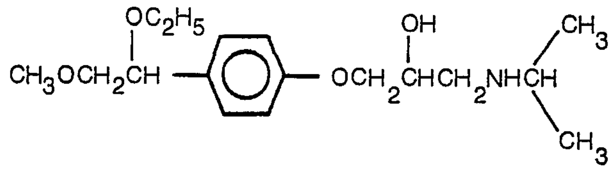

(IX)

In the last step methoxymetoprolol (VIII) and ethoxymetoprolol (IX) are reduced to metoprolol (I), which after this can be converted to e.g. the tartrate salt.

Hydrogenation of the benzyl group is in itself a known reaction (see e.g. Organic Reactions 7 (1953) 263), but never before an alkoxy- or aryloxy group was removed from an $\alpha$-alkylsubstituted alkyl- or arylbenzylether by catalytic hydrogenation. The following reaction was thus not known before:

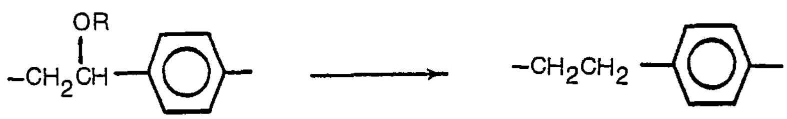

wherein

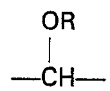

is an ether group.

In the art, however, one case was known, in which a cyclic ether, substituted in the 2-position with a phenyl group, was hydrogenated as follows (J. Am. Chem. Soc. 70 (1948) 1490):

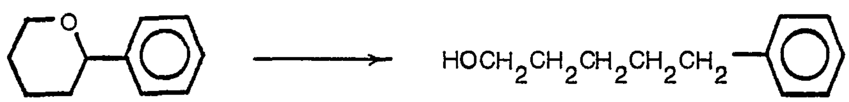

This case differs very much from the case of the invention already because of the ether being cyclic.

In addition to this some reactions are known in the art, in which an alkoxy group was removed from an $\alpha,\alpha$-dialkyl-alkyl-benzylether by catalytic hydrogenation as follows (J. Am. Chem. Soc. 89 (1967) 4233 and Bull, Chem. Soc. Japan 43 (1970) 2143):

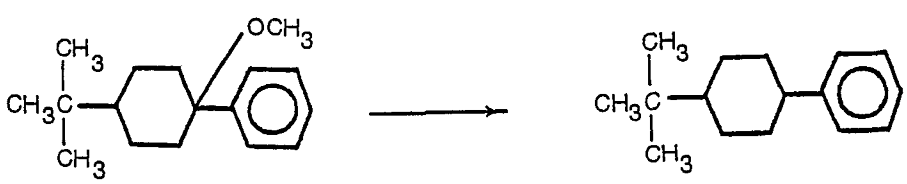

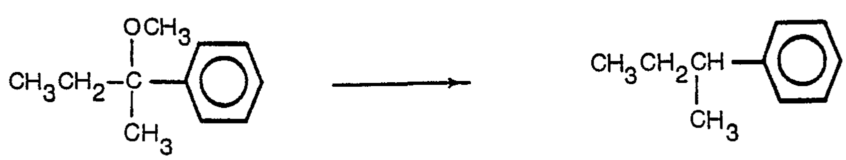

These cases differ from the case of the invention primarily because the benzyl carbon is disubstituted, while it is monosubstituted in this invention. In addition to this Raney-nickel and "palladium-hydride" have been used as catalysts in the above mentioned cases rather than a palladium catalyst, which is preferred for the present invention.

The new process of the invention for the preparation of 1-isopropylamino-3-[4-(2-methoxyethyl)-phenoxy]-2-propanol or metaprolol (I) thus also provides a new process for removing the alkoxy group from an $\alpha$-alkylsubstituted alkylbenzylether by catalytic hydrogenation.

The intermediates of the invention are new compounds, except the hydroxyphenol (III), which has been described in the Finnish application 792950.

The above described two reaction series, the methoxy- and the ethoxy route, are preferably performed all the way to the methoxy- and ethoxymetoprolol (VIII and IX) without isolation of intermediates, which makes the practical accomplishment of the reaction series very easy and simple. Both reaction series also succeed with good yields. The reaction series can, if desired, also be carried out so, that each intermediate or part of the intermediates are isolated. This is of course a much more laborious way than a reaction series without isolation of intermediates, especially on a technical scale.

The reduction of the ketophenolate (II) to hydroxyphenolate (III) is preferably performed with sodium borohydride in a suitable solvent. e.g. water, but preferably in an alcohol because of the succeeding reaction. The reduction is carried out at a temperature of about 30—90°C, which also depends on the solvent used. If one wants to proceed using the methoxy-route, it is most practical to carry out the reduction in methanol.

By adjusting the pH of the reaction mixture after the reduction to the acid side with a suitable acid, e.g. concentrated sulfuric acid, and refluxing the reaction mixture after this, the methoxyphenol (IV) is directly obtained without isolation of the hydroxyphenol (III). The methanol, used as solvent, hereby reacts with the hydroxyphenol. If the intention is to proceed using the ethoxy-route, it is most advantageous to perform the reduction in ethanol and thereafter reflux the mixture correspondingly as above, in order to arrive directly at the ethoxyphenol (V). Then the reaction mixtures are filtered. The methanolic solution is neutralized with e.g. sodium methoxide and the ethanolic solution correspondingly with sodium ethoxide.

In the following step the methoxyphenol (IV) is reacted with epichlorohydrine at alkaline conditions, preferably in the presence of potassium carbonate, in methanolic solution without isolation of the above obtained methoxyphenol (IV). The reaction is performed at the boiling point of the mixture or at a lower temperature. The ethoxyphenol (V) is reacted in a corresponding way in ethanolic solution.

The methoxyepoxide (VI) in methanolic solution and the ethoxyepoxide (VII) in ethanolic solution are then preferably reacted with isopropylamine without changing the solvent either at the boiling point of the mixture or at a lower temperature. Methoxymetoprolol (VIII) and ethoxymetoprolol (IX) are then formed, and these are advantageously purified by extraction at this stage.

In the final step the benzyl ethers, methoxy- and ethoxymetoprolol (VIII and IX), are reduced primarily by catalytic hydrogenation to metoprolol (I). The hydrogenation is preferably performed with a palladium on carbon catalyst in acetic acid in the presence of an acid catalyst, e.g. hydrogen chloride. The reaction proceeds with sufficient speed already at room temperature, and even at 100°C the reaction succeeds. It was very surprising that the reaction occurred so easily at elevated temperature and even more so at room temperature. At least the product can be converted to e.g. the tartrate salt.

The invention is illustrated in more detail by the following examples.

### Example 1

1-Isopropylamino-3-[4-(1,2-dimethoxyethyl)phenoxy]-2-propanol

18,8 g (100 mmol) sodium salt of 4-(methoxyethanoyl)-phenol is dissolved in 120 ml hot methanol. 1,9 g (50 mmol) sodium borohydride is added in small portions while stirring the mixture and keeping the temperature at 50°C. When all of the sodium borohydride has been added the mixture is stirred for an additional hour at 50°C, after which the mixture is cooled. While stirring the reaction mixture and at a temperature of 20°C 4 g concentrated sulfuric acid is added dropwise and after this 50 per cent sulfuric acid solution, until pH reaches the value 3 as determined with pH paper [4-(1-hydroxy-2-methoxyethyl)phenol, m.p. 102—4°C, is formed as intermediate]. Then the mixture is refluxed for one hour. After cooling, the mixture is filtered and neutralized with methanolic solution of sodium methoxide. [4-(1,2-dimethoxyethyl)phenol, m.p. 58—60°C, is formed as intermediate]. 17,3 g (125 mmol) water-free potassium-carbonate is added. Thereafter is added dropwise 20,8 g (225 mmol) of epichlorohydrine while stirring the reaction mixture and at a temperature of 10°C. The mixture is refluxed for 30 min. with continued stirring. [1,2-epoxy-3-[4-(1,2-dimethoxyethyl)phenoxy]-propane, an oil with a b.p. of 145—9°C/0,3 mmHg, is formed as intermediate]. The mixture is cooled and 31,3 g (530 mmol) isopropylamine added. Then the mixture is refluxed for 1 hour. Activated carbon is added and refluxed for another 5 minutes. The cooled solution is filtered, after which the solvent is distilled off. The residue is colourless oily 1-isopropylamino-3-[4-(1,2-dimethoxyethyl)phenoxy]-2-propanol, whose $^1H$—NMR-sprectrum is presented in FIG. 1. The yield is 24,4 g (82%). The melting point of the tartrate is 134—136°C.

### Example 2

1-Isopropylamino-3-[4-(2-methoxyethyl)phenoxy]-2-propanol

24,4 g (82 mmol) 1-isopropylamino-3-[4-(1,2-dimethoxyethyl)phenoxy]-2-propanol, obtained in example 1, is dissolved in 124 ml acetic acid. 12,7 g (123 mmol HCl) conc. hydrochloric acid and 2,44

g 10% Pd on carbon is added. The mixture is hydrogenated at 20°C until the reaction is completed (about 5 hours). The catalyst is removed by filtration after which 19,6 g (185 mmol) solid sodium carbonate is added. The solvent is distilled off. The residue is dissolved in water. The aqueous solution is then washed with toluene, after which pH is adjusted to about 9 with solid $Na_2CO_3$. After this the product is extracted with toluene. The toluene solution is washed with water, then the solvent is evaporated. The residue, 19,0 g (87%), is 1-isopropylamino-3-[4-(2-methoxyethyl)phenoxy]-2-propanol. The product is recrystallized from hexane to give a product with the m.p. 45°C. The melting point of the tartrate, prepared in acetone containing tartaric acid, is about 120°C.

Example 3

1-Isopropylamino-3-[4-(1-ethoxy-2-methoxyethyl)phenoxy]-2-propanol

18,8 g (100 mmol) sodium salt of 4-(methoxyethanoyl)phenol is dissolved in 150 ml hot ethanol (abs.). By stirring and at a temperature of 70°C 1,9 g (50 mmol) sodium borohydride is added in small portions. When all of the sodium borohydride has been added the mixture is stirred for an additional hour at 70°C, after which the mixture is cooled. By stirring the reaction mixture and at a temperature of 20°C 4 g concentrated sulfuric acid and thereafter 50 per cent sulfuric acid-ethanol solution is added dropwise until pH is 3 as determined with pH-paper. [4-(1-hydroxy-2-methoxyethyl)phenol is formed as intermediate, see example 1]. Then the mixture is refluxed for 1 hour.

After cooling, the mixture is filtered and neutralized with an ethanolic sodium ethoxide solution. [As intermediate is formed 4-(1-ethoxy-2-methoxyethyl)phenol, m.p. 66—8°C]. 17,3 g (125 mmol) water-free potassium carbonate is added. By stirring the reaction mixture and at a temperature of 10°C is thereafter added 20,8 g (225 mmol) epichlorohydrine dropwise. The mixture is refluxed for 30 min at continued stirring. [1,2-epoxy-3-[4-(1-ethoxy-2-methoxyethyl)phenoxy]propane, an oil with a b.p. of 148—50°C/0,3 mmHg, is formed as intermediate]. The mixture is cooled and 31,3 g (530 mmol) isopropylamine added. Then the mixture is refluxed for 30 minutes. Activated carbon is added and the refluxing continued for another 5 minutes. The cooled mixture is filtered, after which the solvent is distilled off. The residue is dissolved in toluene and washed with water. The residue is colourless, oily 1-isopropylamino-3-[4-(1-ethoxy-2-methoxyethyl)phenoxy]-2-propanol, whose $^1H$—NMR-spectrum is shown in FIG. 2. The yield is 27,1 g (87%). The melting point of the tartrate is 135—37°C.

Example 4

1-Isopropylamino-3-[4-(2-methoxyethyl)phenoxy]-2-propanol

27,1 g (87 mmol) 1-isopropylamino-3-[4-(1-ethoxy-2-methoxyethyl)phenoxy]-2-propanol, obtained in example 3, is dissolved in 130 ml acetic acid. 13,5 g (131 mmol HCl) conc. hydrochloric acid and 2,71 g 10 per cent palladium on carbon is added. The mixture is hydrogenated at a temperature of 20°C until the reaction is completed (about 5 hours). The catalyst is removed by filtration, after which 20,8 g (196 mmol) solid sodium carbonate is added. The solvent is distilled off. The residue is dissolved in water. The aqueous solution is then washed with toluene, after which pH is adjusted to about 9 with solid $Na_2CO_3$. Then the product is extracted with toluene. The toluene solution is washed with water, after which the solvent is evaporated. The residue, 19,6 g (85%), is 1-isopropylamino-3-[4-(2-methoxyethyl)-phenoxy]-2-propanol. The product is recrystallized from hexane to give a product with a m.p. of 45°C. The melting point of the tartrate, prepared in acetone containing tartaric acid, is about 120°.

## Claims

1. A process for the preparation of the therapeutically active 1-isopropylamino-3-[4-(2-methoxyethyl)-phenoxy]-2-propanol of the formula

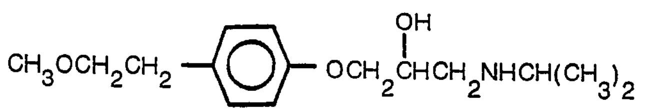

(I)

or an acid addition salt thereof, characterized in that 1-isopropylamino-3-[4-(1-alkoxy-2-methoxyethyl)phenoxy]-2-propanol of the formula

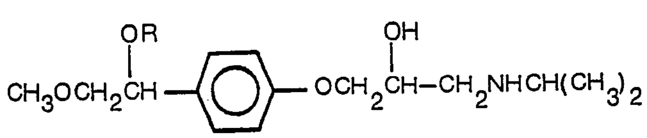

wherein R is a methyl or ethyl group, is hydrogenated catalytically in an organic solvent in the presence of an acid catalyst to 1-isopropylamino-3-[4-(2-methoxyethyl)-phenoxy]-2-propanol (I), which by

conventional methods can be converted to acid addition salts.

2. A process as claimed in claim 1, characterized by reacting 4-(1-hydroxy-2-methoxyethyl)-phenol with methanol or ethanol at acid conditions to 4-(1-alkoxy-2-methoxyethyl)phenol of the formula

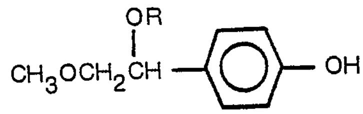

wherein R is a methyl or ethyl group, which is reacted at alkaline conditions with epichlorohydrine to 1,2-epoxy-3-[4-(1-alkoxy-2-methoxyethyl)phenoxy]propane of the formula

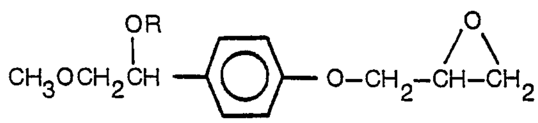

wherein R is the same as before, which in the next step is reacted with isopropylamine to 1-isopropylamino-3-[4-(1-alkoxy-2-methoxyethyl)phenoxy]-2-propanol of the formula

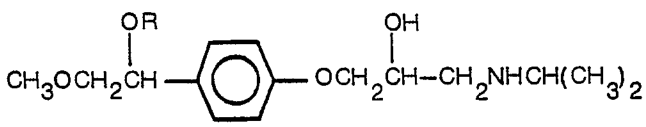

wherein R is the same as before, which is hydrogenated catalytically as stated in claim 1 to 1-isopropylamino-3-[4-(2-methoxyethyl)-phenoxy]-2-propanol, which by conventional methods can be converted to acid addition salts.

3. A process as claimed in claim 2, characterized by reducing the sodium salt of 4-(methoxyethanoyl)phenol to 4-(1-hydroxy-2-methoxyethyl)phenol, which is further processed as stated in claim 2 and claim 1 to 1-isopropylamino-3-[4-(2-methoxyethyl)-phenoxy]-2-propanol, which by conventional methods can be converted to acid addition salts.

4. A process as claimed in claim 1, 2 or 3, characterized thereby that as catalyst in the step of catalytical hydrogenation is used palladium on carbon.

5. A process as claimed in claim 4, characterized thereby, that the solvent in the step of catalytical hydrogenation is acetic acid.

6. A process as claimed in claim 5, characterized thereby, that the acid catalyst in the step of catalytical hydrogenation is hydrogen chloride.

7. An intermediate formed in the preparation of the therapeutically active 1-isopropylamino-3-[4-2-methoxyethyl)phenoxy]-2-propanol characterized by the formula

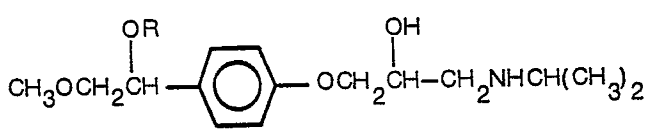

wherein R is the same as before.

**Revendications**

1. Procédé pour la préparation de l'isopropylamino-1 [(méthoxy-2 éthyl)-4 phénoxy]-3 propanol-2, thérapeutiquement actif, de formule:

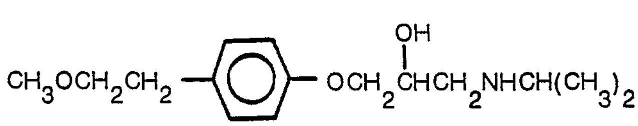

(I)

ou d'un de ses sels d'addition avec un acide, caractérisé en ce qu'on fait une hydrogénation catalytique de l'isopropylamino-1 [(alcoxy-1 méthoxy-2 éthyl)-4 phénoxy]-3 propanol-2 de formule:

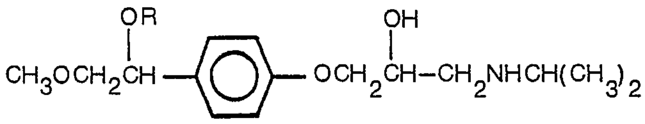

dans laquelle R est un groupe méthyle ou éthyle, dans un solvant organique en présence d'un catalyseur acide, pour obtenir l'isopropylamino-1 [(méthoxy-2 éthyl)-4 phénoxy]-3 propanol-2(I), qui peut être converti par des procédés classiques en ses sels d'addition avec des acides.

2. Procédé selon la revendication 1, caractérisé en ce qu'on fait réagir le (hydroxy-1 méthoxy-2 éthyl)-4 phénol avec le méthanol ou l'éthanol dans des conditions acides, pour obtenir le (alcoxy-1 méthoxy-2 éthyl)-4 phénol de formule:

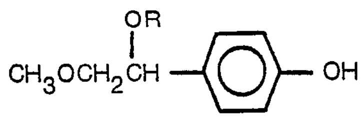

dans laquelle R est un groupe méthyle ou éthyle, qu'on fait réagir dans des conditions alcalines avec l'épichlorhydrine pour obtenir l'époxy-1,2 [(alcoxy-1 méthoxy-2 éthyl)-4 phénoxy]propane de formule:

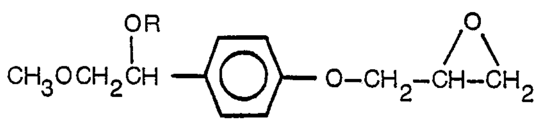

dans laquelle R est le même que ci-dessus, qu'on fait réagir ensuite avec l'isopropylamine pour obtenir l'isopropylamino-1 [(alcoxy-1 méthoxy-2 éthyl)-4 phénoxy]-3 propanol-2 de formule:

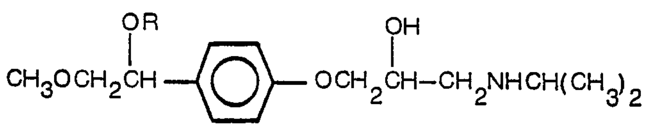

dans laquelle R est le même que ci-dessus, sur lequel on fait une hydrogénation catalytique telle que décrit dans la revendication 1 pour obtenir l'isopropylamino-1 [(méthoxy-2 éthyl)-4 phénoxy]propanol-2, qui peut être converti selon des procédés classiques en des sels d'addition avec des acides.

3. Procédé selon la revendication 2, caractérisé en ce qu'on réduit le sel de sodium du (méthoxy éthyl)-4 phénol en (hydroxy-1 méthoxy -2 éthyl)-4 phénol, qu'on transforme ensuite comme décrit dans les revendications 2 et 1 pour obtenir l'isopropylamino-1 [(méthoxy-2 éthyl)-4 phénoxy]-3 propanol-2 qui peut être converti par des procédés classiques en des sels d'addition avec des acides.

4. Procédé selon la revendication 1, 2 ou 3, caractérisé en ce que comme catalyseur au stade de l'hydrogénation catalytique, on utilise du palladium sur carbone.

5. Procédé selon la revendication 4, caractérisé en ce qu'au stade de l'hydrogenation catalytique, le solvant est l'acide acétique.

6. Procédé selon la revendication 5, caractérisé en ce qu'au stade de l'hydrogénation catalytique, le catalyseur acide est l'acide chlorhydrique.

7. Produit intermédiaire formé dans la préparation de l'isopropylamino-1 [(méthoxy-2 éthyl)-4 phénoxy]-3 propanol-2, caractérisé par la formule:

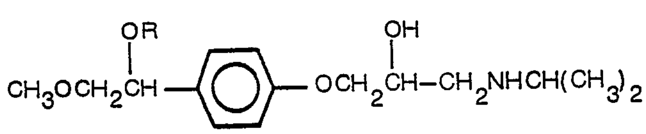

dans laquelle R est le même que ci-dessus.

**Patentansprüche**

1. Verfahren zur Herstellung des therapeutisch aktiven 1-Isopropylamino-3-[4-(2-methoxyäthyl)-phenoxy]-2-propanols der Formel

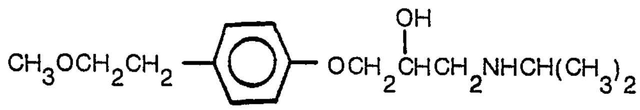

(I)

oder eines Säureadditionssalzes desselben, dadurch gekennzeichnet, daß 1-Isopropylamino-3-[4-(1-alkoxy-2-methoxyäthyl)-phenoxy]-2-propanol der Formel

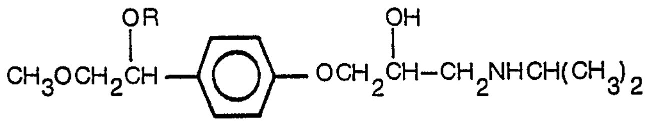

worin R eine Methyl- oder Äthylgruppe bedeutet, in einem organischen Lösungsmittel in Anwesenheit eines Säurekatalysators zu 1-Isopropylamino-3-[4-(2-methoxyäthyl)-phenoxy]-2-propanol (I) katalytisch hydriert wird, welches mittels herkömmlicher Verfahren in Säureadditionssalze umgewandelt werden kann.

2. Verfahren nach Anspruch 1, dadurch gekennzeichnet, daß 4-(1-Hydroxy-2-methoxyäthyl)-phenol mit Methanol oder Äthanol unter sauren Bedingungen zu 4-(1-Alkoxy-2-methoxyäthyl)-phenol der Formel

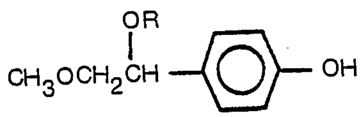

worin R eine Methyl- oder Äthylgruppe bedeutet, umgewandelt wird, welches unter alkalischen Bedingungen mit Epichlorhydrin zu 1,2-Epoxy-3-[4-(1-alkoxy-2-methoxyäthyl)-phenoxy]-propan der Formel

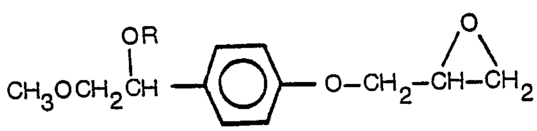

worin R die gleiche Bedeutung wie oben hat, umgesetzt wird, welches im nächsten Schritt mit Isopropylamin zu 1-Isopropylamino-3-[4-(1-alkoxy-2-methoxyäthyl)-phenoxy]-2-propanol der Formel

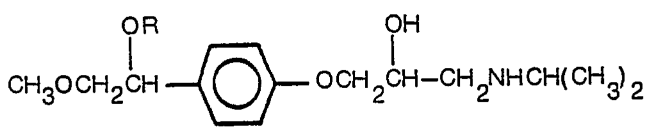

worin R die gleiche Bedeutung hat wie zuvor, umgesetzt wird, welches, wie in Anspruch 1 angegeben, katalytisch zu 1-Isopropylamino-3-[4-(2-methoxyäthyl)-phenoxy]-2-propanol hydriert wird, welches mittels herkömmlicher Verfahren in Säureadditionssalze umgewandelt werden kann.

3. Verfahren nach Anspruch 2, dadurch gekennzeichnet, daß das Natriumsalz von 4-(Methoxyäthanoyl)-phenol zu 4-(1-Hydroxy-2-methoxyäthyl)-phenol reduziert wird, welches, wie in den Ansprüchen 2 und 1 angegeben, zu 1-Isopropylamino-3-[4-(2-methoxyäthyl)-phenoxy]-2-propanol weiterverarbeitet wird, welches mittels herkömmlicher Verfahren in Säureadditionssalze umgewandelt werden kann.

4. Verfahren nach den Ansprüchen 1, 2 oder 3, dadurch gekennzeichnet, daß als Katalysator beim Schritt der katalytischen Hydrierung Palladium auf Kohle verwendet wird.

5. Verfahren nach Anspruch 4, dadurch gekennzeichnet, daß das Lösungsmittel beim Schritt der katalytischen Hydrierung Essigsäure ist.

6. Verfahren nach Anspruch 5, dadurch gekennzeichnet, daß der Säurekatalysator beim Schritt der katalytischen Hydrierung Chlorwasserstoff ist.

7. Zwischenprodukt, hergestellt bei der Herstellung des therapeutisch aktiven 1-Isopropylamino-3-[4-(2-methoxyäthyl)-phenoxy]-2-propanols, gekennzeichnet durch die Formel

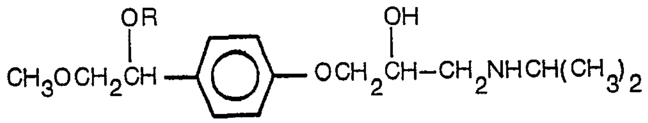

worin R die vorher angegebene Bedeutung hat.

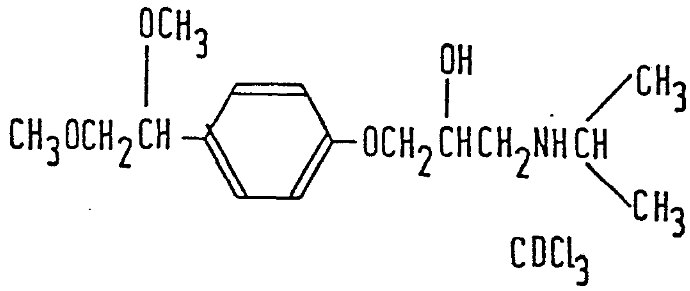

$CDCl_3$

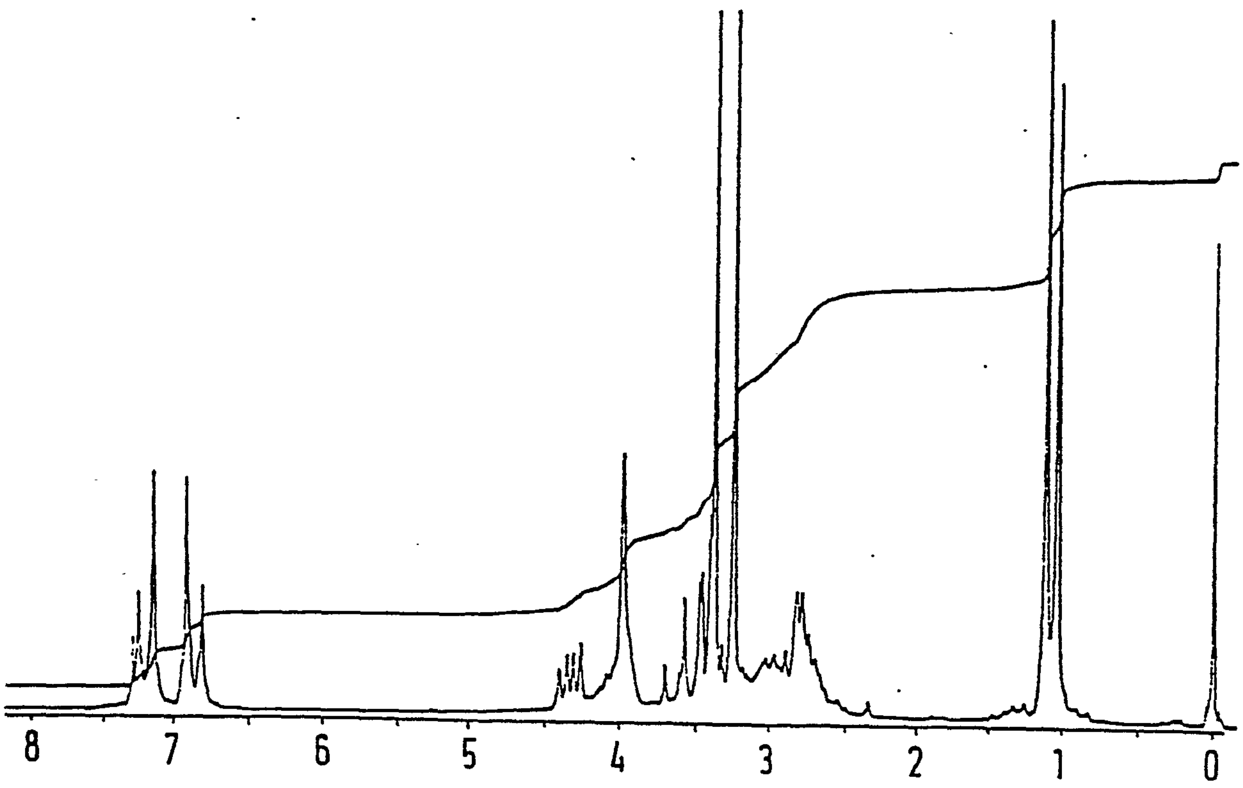


FIG.1

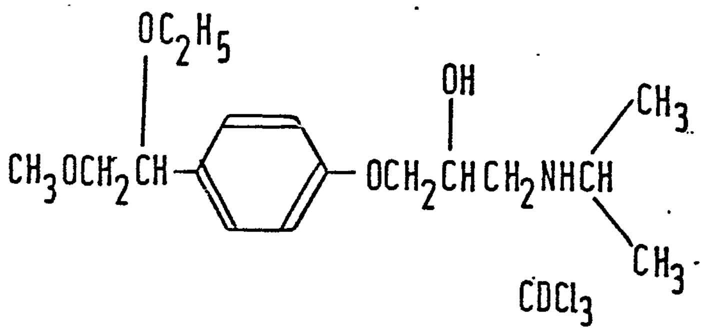

CDCl$_3$

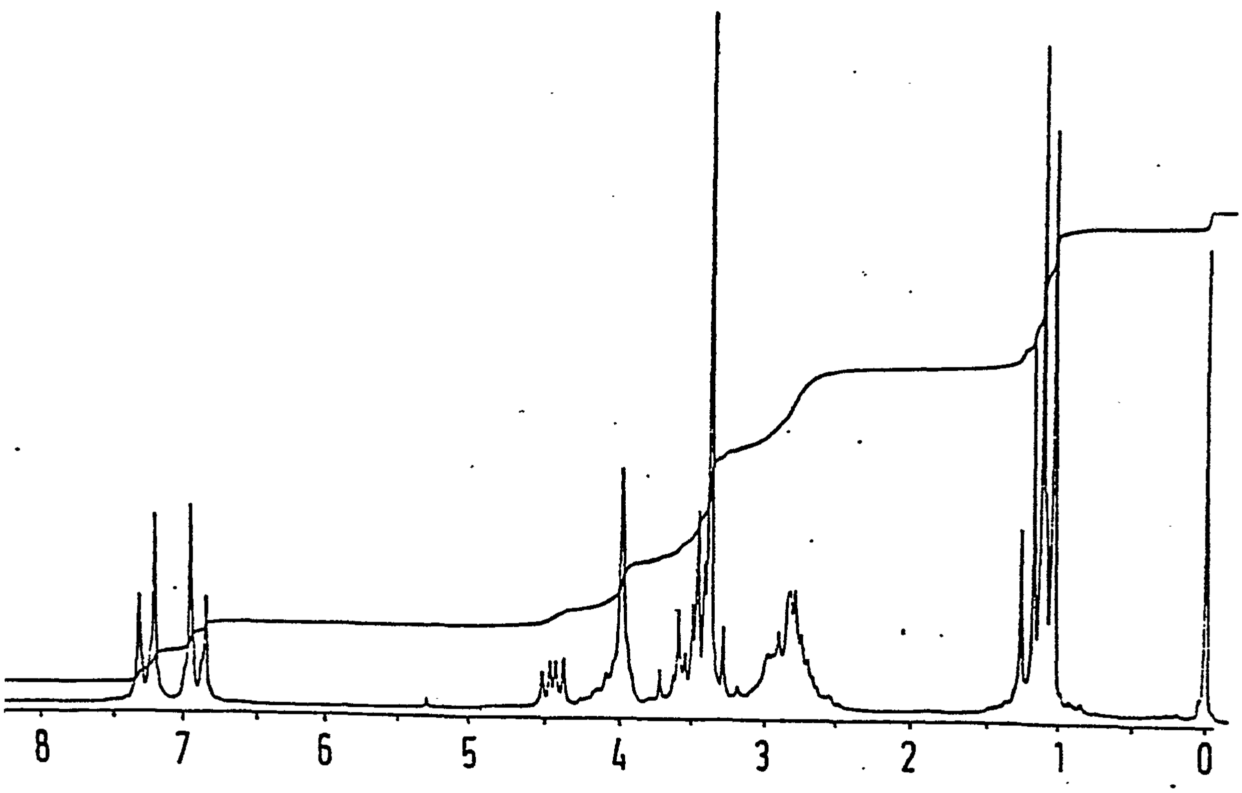


FIG.2